# EUROPÄISCHE PATENTSCHRIFT

(11) **EP 2 242 563 B1**
(45) Veröffentlichungstag und Bekanntmachung des Hinweises auf die Patenterteilung: **22.05.2019**
(21) Anmeldenummer: 08868015.2
(22) Anmeldetag: 30.12.2008
(51) Int. Cl.: B01D 71/22, B01D 69/08, B01D 53/22, B01D 63/02, B01D 69/12, B01D 71/10, B29C 48/05, B29C 48/08

(54) **Verwendung eines Membranfilters zur Aufbereitung von gebrauchtem Dialysat**
Use of a membrane filter for the treatment of used dialysate
Utilisation d'un filtre à membrane pour le traitement de dialysat usagé

(30) Priorität: 03.01.2008 DE 102008003090
(43) Veröffentlichungstag der Anmeldung: 27.10.2010
(73) Patentinhaber: Fresenius Medical Care Deutschland GmbH, 61352 Bad Homburg (DE)
(72) Erfinder: FISLAGE, Rainer, 66606 St. Wendel (DE); RAIKO, Igor, 66606 St. Wendel (DE)
(74) Vertreter: Stolmár & Partner Patentanwälte PartG mbB
(86) Internationale Anmeldenummer: PCT/EP2008/011149
(87) Internationale Veröffentlichungsnummer: WO 2009/083260

(56) Entgegenhaltungen:
- EP-A- 0 682 977
- US-A- 4 978 451
- US-A- 5 085 676
- US-A- 5 505 890
- US-A- 6 013 182
- US-A1- 2006 234 582
- US-A1- 2007 213 665

## Beschreibung

Die vorliegende Erfindung betrifft eine Verwendung in der Hämo- und Peritonealdialyse zur Aufwertung des Dialysats.

Kapillarmembranen unterschiedlicher Zusammensetzungen sind insbesondere aufgrund ihrer zunehmenden Verwendung in der Dialysetechnik bekannt. Die Verwendung und die Herstellung von Membranen, insbesondere von Kapillarmembranen in der Dialysetechnik ist beispielsweise in der Veröffentlichung von Samtleben und Lysaght in: Hörl et al. Replacement of Renal Function by Dialysis 5th ed., Kluwer, 2004, S. 709 bis 724 beschrieben.

Techniken zur Herstellung von Hohlfasermembranen sind beispielsweise in M. Mulder, Basic Principles of Membrane Technology second ed., Kluwer 1996, S. 71-91 offenbart. Typische Verfahren umfassen dabei das so genannte Phaseninversionsverfahren (s.u.), Schmelzspinnverfahren oder das "Dry-Wet-Spinnverfahren" (s. z.B. Hao et al. J. Appl. Polym. Science 62, 129-133 (1996)).

Für die Herstellung von Kapillarmembranen, insbesondere mittels des Phaseninversionsverfahrens, werden oftmals so genannte Hohlfaserdüsen eingesetzt. Bei der Herstellung einer Hohlfasermembran mittels einer Hohlfaserdüse wird die Hohlfasermembran in einem so genannten Fällungsspinnprozess hergestellt, wobei die auszufällenden Polymere aus einem Ringspalt einer Düsenanordnung austreten, während das entsprechende Fällungsmittel aus einer zentralen Fällungsmittelbohrung ausströmt. Eine Hohlfaserdüse der genannten Art ist beispielsweise in der DE 10211051 A1 offenbart.

Komposit-Hohlfasermembranen aufgebaut aus mehreren Schichten unterschiedlicher Funktion sind aus dem Stand der Technik schon bekannt:
Die WO 00/78437 offenbart eine geträgerte Hohlfasermembran bei der die Trägerschicht aus versponnenen Polymerfasern besteht, die der Gesamtfaser eine erhöhte Lebensdauer und Widerstandsfähigkeit gegenüber Abrieb und Zug während ihres Einsatzes in der Mikrofiltration oder Ultrafiltration verleihen. Auf dieser Trägerstruktur ist ein Polymerfilm aufgebracht, in den Teilchen aus kalziniertem Alpha-Aluminiumdioxid dispergiert sind.

Die US 2007/0213665 offenbart eine tragbare Niere umfassend eine Kartusche zur Regenerierung des Dialysats während der Nierendialyse. In der Kartusche ist eine Membran aus einem Kompositmaterial angeordnet, die aus einer Polysulfonlage besteht, auf die ein nicht näher spezifiziertes Celluloseacetat gecoated ist.

Die EP 418 432 A1 offenbart eine geträgerte hydrophile Kompositmembran, bei der Cuproammonium- regenerierte Cellulose auf einer Trägerschicht aus beispielsweise Polypropylen, Polyvinylidenfluorid etc. abgeschieden wird. Cuproammoniumregenerierte Cellulose ist nichtchemisch derivatisierte Cellulose in ihrem natürlichen Zustand. Die Beschichtung der Hohlfasermembran erfolgt nicht innenseitig sondern an ihrer äußeren Oberfläche.

Das US 4,276,172 offenbart eine unbeschichtete Cellulosemembran zur Blutdialyse unter Verwendung von Cuproammonium-Cellulose, enthaltend mindestens eine Dialkylaminocellulose enthaltende Schicht. Probleme ergeben sich hierbei in Bezug auf die Festigkeit des Verbunds der Schichten untereinander. Die Poren der dort beschriebenen Membran sind so groß, dass sie gegenüber niedermolekularen organischen Verbindungen bzw. Kationen mit Harnstoff unspezifisch ist. Die Innenwandstärken der innenseitigen Schicht einer derartigen Membran beträgt 10-50 % der Gesamtwandstärke der Hohlfasermembran.

Das EP 286 091 B1 offenbart eine Polysulfon-Hohlfasermembran, die mit einer Lösung aus Ethylcellulose beschichtet wird zur Verwendung bei der Fluidseparation in industriellen Prozessen.

Das EP 359 834 B1 beschreibt ebenfalls mehrlagig aufgebaute Hohlfasermembranen aus Polysulfon- und Celluloseacetatschichten, wobei Celluloseacetat durch Abscheiden aus Lösung auf die bereits fertige (präformierte) Polysulfonhohlfaser aufgebracht wird zur Verwendung in industriellen Prozessen.

Das US 5,156,740 offenbart des Weiteren eine Kompositmembran bestehend aus einer nicht porösen Trennschicht aus quervernetztem Polyvinylalkohol sowie einer Trägerschicht aus Polysulfon zur Anwendung in Pervaporationsverfahren.

EP0682977 A2 beschreibt eine polymere Gastrennmembran mit einer Trennschicht. US5085676 A offenbart Flüssigkeitstrennungsmembranen, die durch Coextrusion von Polymerlösungen hergestellt werden, um Stütz- und Trennschichten zu bilden. US2006234582 A1 beschreibt Membranen, die für die Hämodialyse geeignet sind. US6013182 A offenbart eine selektiv permeable Hohlfasermembran für die Blutdialyse, die im Wesentlichen aus einem Cellulosederivat besteht. US5505890 A offenbart ein Verfahren zur Herstellung von Celluloseacetatmembranen für die Dialyse.

Bei medizinischen Verfahren wie der Peritoneal- und der Hämodialyse kann das mit urämischen Toxinen beladene Dialysat beispielsweise unter Verwendung von Adsorbermaterialien regeneriert werden, um den Verbrauch von hochreinen Dialysatlösungen zu minimieren, z.B. um tragbare Dialysesysteme bereit zu stellen. Ebenso ist es üblich, dass das Dialysat verworfen wird.

Die im menschlichen Stoffwechsel täglich anfallende Menge von ca. 20 bis 30 g Harnstoff werden zum größten Teil von den eingesetzten Adsorbermaterialien verbraucht. Typischerweise werden entweder in wässriger Phase Kationenaustauscher eingesetzt oder - wie vorstehend im Stand der Technik beschrieben - Hohlfaserkapillarmembranen mit selektiver Permeabilität für Harnstoff, was insbesondere bei tragbaren Dialysesystemen bei der Aufwertung von Dialysat von Vorteil ist (US 2007/0213665 A1). Allerdings führt bei den vorgenannten Systemen eine unbefriedigende Harnstoffselektivität gegenüber ein- und zweiwertigen Kationen zu Konkurrenzreaktionen auf dem der Membran nachfolgend angeordneten Adsorbermaterial. Dies senkt die Adsorberkapazität und erfordert umgekehrt eine höhere Menge an Adsorbermaterial, so dass ein unerwünscht höheres Gewicht des Adsorbermoduls nötig ist.

Die bisher bekannten durch Beschichtung hergestellten Kompositmembranen wiesen weiter den Nachteil auf, dass ihre Herstellung, d.h. insbesondere ihr Aufbau nur durch komplizierte und aufwendige Prozessschritte realisierbar war.

Weiter können bei den aus dem Stand der Technik bekannten Hohlfasermembranen nicht so dünne Schichtdicken der selektiven Schicht, insbesondere der für Harnstoff selektiven Schicht realisiert werden. Damit waren ihrer Selektivität, d.h. Maximierung der Abtrennung der erwünschten Stoffe und Minimierung der mit durch die selektive Schicht hindurchtretenden unerwünschten Verbindungen, Grenzen gesetzt. Insbesondere waren bei den bislang bekannten Komposithohlfasermembranen für die Abtrennung von Harnstoff die Diffusionswege des Harnstoffes zu groß, so dass die Abtrennung unvollständig und langwierig war.

Es bestand daher die Aufgabe, eine Mehrschicht-(Komposit)-Hohlfaserkapillarmembran zur Verfügung zu stellen, die insbesondere bei der selektiven Abtrennung von Harnstoff gegen geladene Verbindungen, wie z.B. Kationen aus Lösungen, von Vorteil ist. Insbesondere sollte die Membran eine selektive Abtrennung von Harnstoff gegenüber ein- oder zweiwertigen Metallkationen, d.h. insbesondere für den menschlichen Organismus essentielle Alkali- und Erdalkalikationen, aufweisen. Weiter sollte diese Membran insbesondere geringe Schichtdicken der Selektionsschicht aufweisen, um die Diffusionswege der abzutrennenden Substanz zu minimieren und damit die Effizienz der Abtrennung der Substanz, insbesondere z.B. Harnstoff erhöhen.

Die Aufgabe der vorliegenden Erfindung wird gelöst durch eine Verwendung nach Anspruch 1.

Der Begriff "Koextrudat" bedeutet, dass Trägerschicht und Selektionsschicht gleichzeitig durch ein dem Fachmann an sich bekanntes Koextrusionsverfahren hergestellt worden sind und beide Schichten zusammen einen festen Verbund (Komposit) bilden.

Das Koextrudat aus der Trägerschicht und der Selektionsschicht erlaubt die gleichzeitige Herstellung der Träger- und der Selektionsschicht in einem einzigen Verfahrensschritt und führt zu einem mechanisch festen Verbund zwischen der Trägerschicht und der Selektionsschicht.

Der Begriff "Selektionsschicht" bedeutet, dass diese Schicht für mindestens einen ausgewählten Stoff aus einem (flüssigen) Stoffgemisch selektiv durchlässig ist und für andere Stoffe des Stoffgemisches undurchlässig ist.

Außerdem ermöglicht der Einsatz eines Koextrudats die Ausbildung extrem dünner Schichten von unter 800 nm. Die Effizienz der Abtrennung wird dadurch erhöht. Die dünnen Schichten gemäß der vorliegenden Erfindung führen dazu, dass die Diffusionswege der abzutrennenden Verbindungen minimiert werden können. Die Wandstärke der Selektionsschicht beträgt dabei bevorzugt 2-5 % der Gesamtwandstärke der Hohlfasermembran.

In bevorzugten Ausführungsformen der Erfindung ist die Selektionsschicht harnstoffselektiv, d.h. nur für Harnstoff durchlässig, insbesondere gegenüber Alkali- und Erdalkalimetallionen wie Na⁺, K⁺, Ca²⁺, Mg²⁺, etc., die diese Schicht nicht passieren können, so dass die Hohlfasermembran besonders bevorzugt in der Hämo- und Peritonealdialyse zur Dialysatregeneration verwendet werden kann. Es versteht sich dabei, dass geringste, unterhalb oder an der Nachweisgrenze liegende Mengen dieser Kationen auch hindurch diffundieren können.

Da durch eine hohe Harnstoff-Membranselektivität geringere Mengen an Adsorber notwendig sind, werden auch wesentliche Gewichtsvorteile beim Einsatz der Membran beispielsweise in Mikrofiltrationssystemen in tragbaren Dialysevorrichtungen erzielt.

Die Dicke der - insbesondere harnstoffselektiven - Selektionsschicht liegt bevorzugt im Bereich von 200 bis 800 nm, insbesondere von 300 bis 600 nm, ganz besonders bevorzugt beträgt die Schichtdicke ca. 500 nm, so dass die Diffusionswege, z.B. von Harnstoff oder anderer ungeladener Verbindungen minimiert werden können, da dadurch die Transportrate des Harnstoffes optimiert wird.

Die Wandstärke der Selektionsschicht richtet sich nach zwei gegenläufigen Bedingungen. Eine hohe Selektivität wird durch eine größere Dicke der selektiven Schicht verursacht. Gleichzeitig wird mit der Dicke der Selektivschicht aber auch die Diffusionsstrecke größer, so dass der Trennprozess verlangsamt und weniger effektiv wird. Erfindungsgemäß liegt die optimale Schichtdicke daher in dem vorstehend genannten Bereich, so dass weder Selektivität noch Diffusion zu stark eingeschränkt sind.

Die Selektionsschicht besteht aus einer Acetylcellulose. Als Acetylcellulose werden typischerweise Celluloseester bezeichnet, die technisch durch Umsetzung von Zellstoff mit Essigsäureanhydrid in Essigsäure oder Methylenchlorid unter Einsatz von starken Säuren in diskontinuierlichen Verfahren hergestellt werden. Typischerweise entstehen dabei zunächst vollständig acetylierte Produkte (Triacetate mit einem Gehalt an Acetyl-Gruppen bzw. gebundener Essigsäure von 44,8 bzw. 62,5 %). Es können auch Ester mit anderen Acylresten, wie z.B. Propionyl- oder Butyrylester, verwendet werden. Ebenso können in bevorzugten Ausführungsformen Mischester mit unterschiedlichen Acylresten, wie Acetyl-, Propionyl-, Butyryl-, längerkettigen oder verzweigten Acylresten verwendet werden. Beispielhaft seien Acetyl-Butyrylcellusloseester oder Propionylbutyrylcelluloseester erwähnt.

Gleichzeitig mit der Acetylierung erfolgt eine säurekatalysierte Depolymerisation des Celluloserückgrates, so dass die typischerweise verwendete Cellulose nur Polymerisationsgrade von ca. 100 bis 350 aufweist.

Die Acetylcellulosen weisen Acylierungs- bzw. Veresterungsgrade von 0,5 bis 3 auf, ganz bevorzugt von 2 bis 3. Ein Acylierungsgrad von 3 entspricht dabei z.B. dem Cellulosetriacetat, ein Acylierungsgrad von 2 entspricht z.B. dem Cellulosediacetat. Der mittlere Acylierungsgrad gibt an, wie viel Acylreste pro Wiederholungseinheit im Mittel an den freien OH-Gruppen der Cellulose gebunden sind. Bevorzugt sind hohe Acylierungsgrade bzw. Veresterungsgrade bis hin zum theoretisch maximal möglichen Acylierungsgrad von 3, da gefunden wurde, dass die Selektivität der insbesondere harnstoffselektiven Schicht mit dem Grad der Acylierung bzw. Veresterung ansteigt. Es wurde dabei gefunden, dass höhere Substitutionsgrade bis z.B. hin zum z.B. Cellulosetriacetat die Selektivität der Acylcelluloseschicht für Harnstoff weiter erhöhen. Dasselbe trifft ebenfalls auf die entsprechenden vorgenannten Mischester zu.

Die Selektionsschicht, bevorzugt die Acetylcelluloseschicht oder Mischestercelluloseschicht, weist typischerweise eine Harnstoffdurchlässigkeit im Bereich von 10 bis 80 g pro Tag und m² auf, ganz besonders bevorzugt zwischen 11 bis 60 g pro Tag und m². Natriumdurchlässigkeiten, das heißt Durchlässigkeiten für einwertig geladene Kationen, weisen Durchlässigkeiten zwischen 0 bis 112 mmol pro Tag und m² auf. Die erfindungsgemäß verwendete Selektionsschicht ist für zweiwertige Kationen, wie z.B. Ca²⁺, Mg²⁺ etc. im Rahmen der üblichen Messgenauigkeit undurchlässig. Die Selektionsschicht ist typischerweise eine dichte, porenfreie Schicht. Unter porenfrei ist in diesem Zusammenhang zu verstehen, dass die Selektionsschicht gegenüber höhermolekularen Substanzen aufgrund ihres Volumens eine Ausschlussgrenze aufweist. Bevorzugt ist diese Ausschlussgrenze schon bei möglichst niedrigen Volumina wirksam, so dass nur monomolekulare Substanzen aufgrund ihrer Größe in der Lage sind, in die Selektionsschicht einzudringen.

Es wurde vorliegend gefunden, dass sich die Natriumchloridpermeabilität bzw. generell die Permeabilität für einwertige Kationen mit der Änderung des Acylierungsgrades bzw. Veresterungsgrades ändert. So wird beispielsweise mit steigendem Veresterungsgrad auch eine Verbesserung der Natriumrückhaltung beobachtet.

Die erfindungsgemäß vorliegenden extrem dünnen Schichten der selektiven Schicht sind mechanisch instabil, so dass eine Trägerschicht erforderlich ist. Diese und das Vorliegen als Koextrudat führen zu einer erhöhten mechanischen Festigkeit der Komposithohlfasermembran verglichen mit den bekannten Kompositmembranen des Standes der Technik.

Das Material der Trägerschicht ist bevorzugt ausgewählt aus Polyvinylpyrrolidon (PVP), Polyethersulfon (PES), Polyetherimid (PEI) Polyamid (PA), Polycarbonat (PC), Polystyrol (PS), Polymethylmethacrylat (PMMA), Polyvinylidenfluorid (PVDF), Polyacrylnitril (PAN), Polyimid (PI), Polysulfon (PSU) und/oder Polyurethan (PU) und deren Mischungen. Beispielsweise ist PVP in bevorzugten Ausführungsformen der Erfindung oft als hydrophilisierender Bestandteil in der Trägerschicht enthalten.

Wichtig ist bei der Auswahl des Materials der Trägerschicht, dass eine ausreichend hohe Permeabilität und Hydrophilie der Trägerschicht vorliegt, so dass entlang der vergleichsweise langen Transportstrecke durch die Trägerschicht kein oder nur ein geringer Diffusionswiderstand durch die hindurchtretende Verbindung, z.B. Harnstoff hervorgerufen wird.

Bevorzugtes Material der Trägerschicht ist erfindungsgemäß Polysulfon, Polyvinylpyrrolidon und deren Mischungen, da die Bedingungen zur Herstellung von beispielsweise Polysulfonmembranen ausreichend gut untersucht sind und sich unterschiedlich hohe Permeabilitäten gezielt durch bekannte Verfahrensparameter einstellen lassen. Ganz besonders bevorzugt ist daher Polysulfon, gegebenenfalls mit einem Zusatz an PVP, das sich aufgrund seiner guten thermodynamischen Kompatibilität beispielsweise mit Polyurethan zu Faserbündeln (Modul) für Mikrofiltrationssysteme vergießen lässt.

Die Dicke der Trägerschicht liegt typischerweise im Bereich von 20 bis 50 µm, bevorzugt im Bereich von 30 bis 40 µm, was, wie vorstehend schon ausgeführt, besonders gut mit Polysulfon erreicht werden kann.

Typische Werte für den Innendurchmesser der Hohlfaserkapillarmembran betragen 20 µm bis 1 mm und die gesamte Wandstärke der Hohlfaserkapillarmembran 20 bis 100 µm.

Ein Verfahren zur Herstellung einer Hohlfasermembran umfassend die Schritte des
a) Bereitstellens zweier Spinnmasselösungen A und B, wobei die Spinnmasselösung A eine Lösung einer veresterten Cellulose ist und die Spinnmasselösung B eine Lösung enthaltend ein Polymer ausgewählt aus der Gruppe bestehend aus Polyvinylpyrrolidon (PVP), Polyethersulfon (PES), Polyetherimid (PEI) Polyamid (PA), Polycarbonat (PC), Polystyrol (PS), Polymethylmethacrylat (PMMA), Polyvinylidenfluorid (PVDF), Polyacrylnitril (PAN), Polyimid (PI), Polysulfon (PSU) und/oder Polyurethan (PU) und deren Mischungen ist;
b) Einstellens der Fällbadtemperatur auf 40 bis 95 °C;
c) In-Kontakt-Bringens der Spinnmasselösungen A und B über eine Hohlfaserdüse mit einem inneren Fällungsmittel; und
d) Koagulierens und Fällens des Extrudats bestehend aus den in den Spinnmasselösungen A und B gelösten Stoffen.

Durch die Verwendung des Spinnprozesses können insbesondere die Dicke des Koextrudats bzw. der beiden das Koextrudat bildenden Schichten gezielt eingestellt werden, so dass eine hohe Harnstoffpermeabilität für die Selektionsschicht sowie weiter eine gute Rückhaltung für ein- bzw. zweiwertige Kationen erzielt wird und gleichzeitig die Trägerschicht so dünn ausgebildet werden kann, dass sich kein oder nur ein geringer Diffusionswiderstand durch den hindurchtretenden Harnstoff bei der Filtration aufbaut.

Dies lässt sich durch den vorstehend genannten Phaseninversionsprozess des Spinnverfahrens besonders gut erzielen. Wie schon gesagt, besteht das Material der Trägerschicht aus Polysulfon, Polyvinylpyrrolidon bzw. deren Mischungen. Ganz besonders bevorzugt besteht das Material der Trägerschicht aus Polysulfon.

In bevorzugten Ausführungsformen des Verfahrens beträgt die Viskosität der Spinnmasselösung A, die Celluloseacetat enthält, 10.000 bis ca. 17.000 mPas (bestimmt mittels eines Haake Rotationsmikrometers (VTSSO) und dem Messbechersystem (MV-ST)). Die Viskosität wird typischerweise durch einen Gehalt von 25 bis 40 Gew.-% Celluloseacetat in beispielsweise Dimethylacetamid erhalten.

Die Viskosität der Spinnmasselösung B, die das Polymer für die Trägerschicht enthält, liegt typischerweise im Bereich von 7.000 bis 13.000 mPas.

Bevorzugt wird als inneres Fällungsmittel im Verfahren Wasser verwendet bei einer Spinngeschwindigkeit von 200 bis 400 mm/s.

Der Begriff "inneres Fällungsmittel" bezeichnet dabei das lumenseitige Fällungsmittel. Erfindungsgemäß wird dabei Wasser verwendet und im Fällbad selbst wird ebenfalls Wasser als Fällungsmittel verwendet. Wasser fungiert als so genanntes "hartes" Fällungsmittel, was dazu führt, dass die Membran an der Innenseite eine erhöhte Undurchlässigkeit gegenüber ein- oder zweiwertigen Kationen, wie z.B. Natrium, Kalium, Magnesium oder Calcium aufweist. Durch die Verwendung eines Luftspaltes zwischen Block und Wasseroberfläche sowie eines sehr langsamen Wassertransports durch die z.B. Celluloseacetatinnenschicht findet in der Außenschicht eine so genannte "weichere" Fällung statt, so dass auf der Außenseite Poren entstehen. Typischerweise erfolgt die Fällung so, dass mit Wasser von außen nach innen durchgefällt wird, wobei ein Porengradient von innen (typischerweise keine Poren) nach außen (große Poren) erhalten wird.

Ohne Luftspalt und Fällen beispielsweise in einem lösungsmittelhaltigem Fällungsbad würde eine Hohlfaser erhalten werden, die von innen und außen gleichzeitig gefällt wurde, so dass die größten Poren in der Mitte der Faser entstehen würden, was für den vorliegenden Zweck der Hohlfasermembran unerwünscht ist_{.}

Die Fällbadtemperatur wird bevorzugt auf eine Temperatur von 40 bis 95 °C, bevorzugt ca. 40 °C, da damit ein Koextrudat erhalten wird, das eine Selektionsschicht aufweist, die eine erhöhte Rückhaltefähigkeit für ein- bzw. zweiwertige Kationen aufweist und noch eine extrem hohe Harnstoffpermeabilität aufweist. Die Blocktemperaturen liegen im Bereich von 5 bis 40 °C.

Ferner wird ein Membranfilter beschrieben, der eine Mehrzahl von Hohlfasermembranen umfasst, wie er beispielsweise in der DE 10 2004 020 226 A1 ganz allgernein beschrieben ist.

Die Erfindung ist anhand von nachstehenden Abbildungen und anhand von Beispielen näher erläutert.

Es zeigen
- Abbildung 1:: eine REM Aufnahme eines Kryobruchs durch eine zweilagige Kompositfaser bestehend aus einem Koextrudat;
- Abbildung 2:: eine Vergrößerung der REM Aufnahme des Kryobruchs aus Abb. 1.

### Beispiel 1:

Eine Hohlfaser wird nach dem so genannten Phaseninversionsverfahren hergestellt. Zunächst werden zwei Spinnmasselösungen A und B hergestellt. Die erste Spinnmasselösung A enthält das Material für die lumenseitige Selektionsschicht der Hohlfasermembran und die zweite Spinnmasselösung B das Material für die Trägerschicht.

Die Spinnmasselösung für die Trägerschicht (die äußere Schicht) besteht aus 20 Gew.-% Udel 3500 Polysulfon und 5 Gew.-% Polyvinylpyrrolidon K90 sowie 1 Gew.-% Wasser, die in Dimethylacetamid gelöst sind. Die Viskosität dieser Lösung betrug ca. 11.500 mPas.

Die Spinnmasse für die lumenseitige Selektionsschicht bestand aus 30 Gew.-% Cellulosediacetat mit einem Molekulargewicht von 29 kD und einem Acetylgehalt von 40 % (erhältlich von der Firma Sigma/Aldrich). Diese wurde unter Rühren in Dimethylacetamid gelöst. Die Viskosität dieser Lösung betrug ca. 15.000 mPas.

Beide Spinnmasselösungen wurden in einem geeigneten Volumenverhältnis durch eine Komposithohlfaserdüse versponnen, wie sie aus dem Stand der Technik bekannt ist. Dabei wurden beide Lösungen durch zueinander konzentrische Düsenkanäle geführt, die die Koextrusion der inneren und äußeren Spinnmasse erlauben. Die beiden konzentrischen Düsenkanäle umgeben einen axialen Kanal, durch den ein Fällungsmittel zugeführt wird, das zur Koagulation der beiden Spinnmasseschichten dient. Als inneres Fällungsmittel wurde Wasser verwendet.

Die Temperatur des Düsenblocks (Spinnblock) lag bei 20 °C, kann jedoch im Rahmen des Verfahrens weiter variiert werden.

Es wurde überraschenderweise gefunden, dass bei tiefer Temperatur ersponnene Fasern (< 30 °C) eine höhere Selektivität von Harnstoff gegenüber Kationen wie Natrium, Kalium, also einwertigen Kationen aufweisen.

Nach dem Austritt aus dem Spinnblock durchlief die Hohlfaser einen Luftspalt von ca. 250 mm bevor sie in ein wassergefülltes Fällbad mit einer Temperatur von ca. 42 °C eintauchte. Anschließend wurde die so erhaltene Komposithohlfaser in einem Spülbad gespült, welches auf 75 °C temperiert wurde. Die Vorschubgeschwindigkeit der Faser betrug 250 mm/s.

Die so erhaltene Hohlfaser wurde anschließend bei ca. 95 °C getrocknet.

Die Volumina von Fällbad und Spülbad und die Vorschubgeschwindigkeit wurden so eingestellt, dass eine lösemittelfreie regelmäßige Hohlfaser erhalten wurde.

Anschließend wurde die trockene Faser gehaspelt. Ein Bündel der Hohlfaser besteht aus 2300 Fasern mit einer Gesamtfläche von 0,4 m². Der Faserinnendurchmesser betrug 200 µm. Der Faseraußendurchmesser betrug 261 µm.

Die Dicke der Selektionsschicht betrug ca. 500 nm.

Die Fasern wurden in ein Gehäuse eingeformt und mit Polyurethan so zu einem Modul vergossen, dass eine unabhängige Anströmung von Faserlumen und Faseraußenseite sichergestellt war.

Derartige Module sind dem Fachmann typischerweise aus der Hämodialyse bekannt.

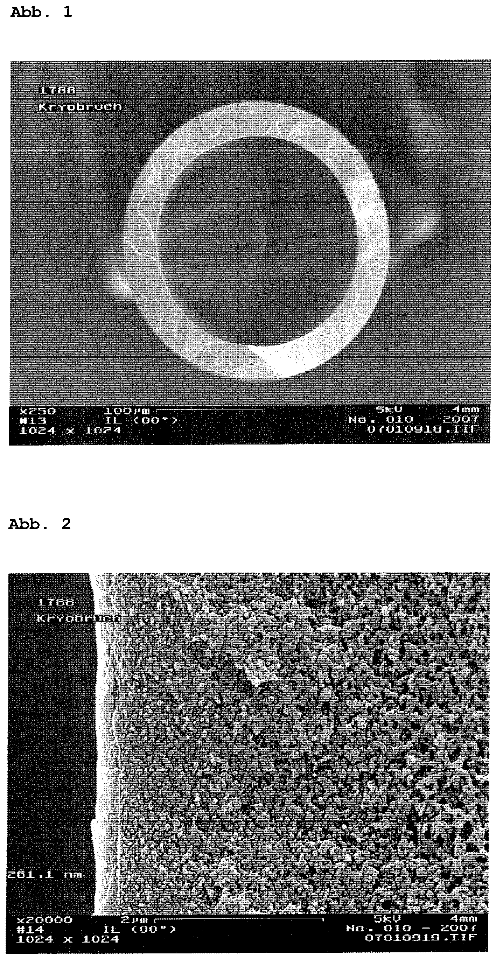

Abb. 1 zeigt eine REM-Aufnahme in 250-facher Vergrößerung und Abb. 2 einen in 20.000-facher Vergrößerung erhaltenen Ausschnitt der Abb. 1.

Der Begriff "Kryobruch" bedeutet, dass die Hohlfasermembran in flüssigen Stickstoff getaucht und anschließend von Hand in Querrichtung gebrochen wurde.

Aus Abb. 2 ist die poröse Struktur der rechtsseitig dargestellten Trägerschicht aus Polysulfon klar erkennbar sowie die nahezu porenfreie Struktur der dünnen, linksseitig dargestellten Selektionsschicht aus Cellulosediacetat.

### Beispiel 2:

### Bestimmung der wesentlichen physikalischen Parameter einer Membran

Die in Beispiel 1 erhaltene Hohlfasermembran wurde anschließend in Bezug auf ihre Ultrafiltrationsrate sowie ihre Durchlässigkeit für Harnstoff und verschiedene Salze untersucht.

Zur Bestimmung der wässrigen Ultrafiltration wurde lumenseitig bei einer Temperatur von 37 °C ein Überdruck angelegt und die Menge an Wasser bestimmt, die von der Lumenseite der Hohlfaser auf die Außenseite der Hohlfaser übertritt.

Die gemessenen Ultrafiltrationsraten der Membran aus Beispiel 1 lagen im Bereich von 0,1 bis 0,3 [ml/h Torr m²].

Zur Bestimmung der Harnstoff- und Salzpermeabilität wurden 500-700 ml einer harnstoffhaltigen Salzlösung verwendet, die 25 mM Harnstoff, 141 mM NaCl, 2,5 mM CaCl₂, 249 mM Glucose enthielt und die lumenseitig mit 50 ml/min durch die Hohlfaser rezirkuliert wurde.

Die Lösung auf der Lumenseite der Hohlfaser befand sich dabei in einem druckdicht abgeschlossenem Behältnis, so dass sich das Volumen der Testlösung über die Versuchszeit nicht ändern konnte.

Auf der Außenseite der Membran wurde im Gegenstrom eine 538 mM Glucoselösung mit einem Durchfluss von 50 ml/min im Gegenstrom gepumpt.

Nach zwei Stunden bei Raumtemperatur wurde eine Probe aus der lumenseitig zirkulierenden Lösung entnommen und mit einem kommerziellen Analysegerät (Cobas Integra 400, Fa. Roche) untersucht.

Aus den Konzentrationen der untersuchten Ausgangslösung kann die Permeabilität und Selektivität der Membran berechnet werden.

Mit der Membran aus Beispiel 1 wurden bei der Abtrennung der vorgenannten harnstoffhaltigen Lösung folgende Ergebnisse erhalten:

**Tabelle 1: Permeabilität und Selektivität der Membran gemäß Beispiel 1**

| | **Natrium** | **Harnstoff** | **Kalzium** |
|---|---|---|---|
| **Anfangswert [mM]** | 158 | 25 | 2,8 |
| **Wert nach 2 h [mM]** | 157 | 15 | 3,0 |

Der Variationskoeffizient der Messung lag bei 1 % für Natrium, 3,5 % für Kalzium und 1,8 % für Harnstoff.

Wie aus den Messungen ersichtlich ist, wird Harnstoff durch die Hohlfasermembran gut abgetrennt, wohingegen Natrium und Kalzium weitgehend zurückgehalten werden.

### Beispiel 3:

Zur weiteren Charakterisierung der Membran wurden Permeationsversuche mit reinen Gasen durchgeführt. Dazu wurde die Hohlfaser lumenseitig mit einem Überdrück von ca. 1 bar des Gases beaufschlagt und der daraus resultierende Gasfluss über die Membran gemessen. Ein typisches Ergebnis zeigt die folgende Tabelle.

**Tabelle 2: Gasfluss durch die Membran bei Raumtemperatur und einem Druckgradienten über die Membran von 1 bar.**

| | **Stickstoff** | **Kohlendioxid** |
|---|---|---|
| **Gasfluss [ml/h Torr m²]** | 0,1 | 15 |

Diese Ergebnisse zeigen, dass die Membran nur sehr wenige Poren aufweist, da übliche Durchflüsse bei herkömmlichen Membranen typischerweise mehrere Liter betragen.

## Patentansprüche

1. Verwendung eines Membranfilters zur Aufbereitung von gebrauchtem Dialysat, umfassend eine Mehrzahl von Hohlfasermembranen hergestellt nach folgendem Verfahren:
a) Bereitstellen zweier Spinnmasselösungen A und B, wobei die Spinnmasselösung A eine Lösung einer veresterten Cellulose für die lumenseitige Selektionsschicht ist und die Spinnmasselösung B eine Lösung enthaltend ein Polymer für die Trägerschicht ausgewählt aus der Gruppe bestehend aus Polyvinylpyrrolidon (PVP), Polyethersulfon (PES), Polyetherimid (PEI), Polyamid (PA), Polycarbonat (PC), Polystyrol (PS), Polymethylmethacrylat (PMMA), Polyvinylidenfluorid (PVDF), Polyacrylnitril (PAN), Polyimid (PI), Polysulfon (PSU), und/ oder Polyurethan (PU) und deren Mischungen,
b) Einstellen der Fällbadtemperatur auf 40 bis 95°C,
c) In-Kontakt-Bringen der Spinnmasselösungen A und B über eine Hohlfaserdüse mit Wasser als innerem Fällungsmittel,
d) Koagulieren und Fällen des Extrudats bestehend aus den in den Spinnmasselösungen A und B gelösten Stoffen, wobei die Spinnblocktemperatur auf 5 bis 40°C eingestellt wird,
wobei die Hohlfasermembranen ein Koextrudat aus einer Trägerschicht und einer lumenseitigen Selektionsschicht umfassen, wobei die Selektionsschicht aus Acetylcellulose besteht und der Acetylierungsgrad im Bereich von 0,5 bis 3 liegt und wobei die Dicke der Selektionsschicht weniger als 800 nm beträgt.

2. Verwendung eines Membranfilters zur Aufbereitung von gebrauchtem Dialysat, umfassend eine Mehrzahl von Hohlfasermembranen nach Anspruch 1, **dadurch gekennzeichnet, dass** die Dicke der Selektionschicht 100 nm bis weniger als 800 nm beträgt.

3. Verwendung eines Membranfilters zur Aufbereitung von gebrauchtem Dialysat, umfassend eine Mehrzahl von Hohlfasermembranen nach Anspruch 1 bis 2, **dadurch gekennzeichnet, dass** die Harnstoffdurchlässigkeit zwischen 10 und 80 g pro Tag und m² und die Natriumdurchlässigkeit zwischen 0 und 112 mmol pro Tag und m² beträgt, gemessen bei einem Durchfluss von 50 ml/min gegen eine Glucoselösung von 538 mM bei Raumtemperatur nach 2 Stunden Zirkulation, wobei zur Bestimmung der Harnstoff- und Salzpermeabilität 500-700 ml einer harnstoffhaltigen Salzlösung verwendet werden, die 25 mM Harnstoff, 141 mM NaCl, 2,5 mM CaCl₂, 249 mM Glucose enthält und die lumenseitig mit 50 ml/min durch die Hohlfaser rezirkuliert wird und wobei auf der Außenseite der Membran im Gegenstrom eine 538 mM Glucoselösung mit einem Durchfluss von 50 ml/min im Gegenstrom gepumpt wird.

4. Verwendung eines Membranfilters zur Aufbereitung von gebrauchtem Dialysat, umfassend eine Mehrzahl von Hohlfasermembranen nach einem der Ansprüche 1 bis 3, **dadurch gekennzeichnet, dass** das Material der Trägerschicht ausgewählt ist aus Polyvinylpyrrolidon (PVP), Polyethersulfon (PES), Polyetherimid (PEI), Polyamid (PA), Polycarbonat (PC), Polystyrol (PS), Polymethylmethacrylat (PMMA), Polyvinylidenfluorid (PVDF), Polyacrylnitril (PAN), Polyimid (PI), Polysulfon (PSU), und/ oder Polyurethan (PU) und deren Mischungen.

5. Verwendung eines Membranfilters zur Aufbereitung von gebrauchtem Dialysat, umfassend eine Mehrzahl von Hohlfasermembranen nach Anspruch 4, **dadurch gekennzeichnet, dass** das Material der Trägerschicht ausgewählt ist aus Polysulfon (PSU), Polyvinylpyrrolidon (PVP) und deren Mischungen.

6. Verwendung eines Membranfilters zur Aufbereitung von gebrauchtem Dialysat, umfassend eine Mehrzahl von Hohlfasermembranen nach einem der Ansprüche 1 bis 5, **dadurch gekennzeichnet, dass** die Dicke der Trägerschicht im Bereich von 20 bis 50 µm, bevorzugt im Bereich 30 bis 40 µm liegt.

7. Verwendung eines Membranfilters zur Aufbereitung von gebrauchtem Dialysat, umfassend eine Mehrzahl von Hohlfasermembranen nach einem der vorhergehenden Ansprüchen, **dadurch gekennzeichnet, dass** die Spinngeschwindigkeit 200 bis 400 mm/s beträgt.

8. Verwendung eines Membranfilters zur Aufbereitung von gebrauchtem Dialysat, umfassend eine Mehrzahl von Hohlfasermembranen nach einem der vorhergehenden Ansprüchen, **dadurch gekennzeichnet, dass** die Viskosität der Spinnmasselösung A, die Celluloseacetat enthält, 10.000 bis 17.000 mPas beträgt.

9. Verwendung eines Membranfilters zur Aufbereitung von gebrauchtem Dialysat umfassend eine Mehrzahl von Hohlfasermembranen nach einem der vorhergehenden Ansprüchen, **dadurch gekennzeichnet, dass** die Viskosität der Spinnmasselösung B, die das Polymer für die Trägerschicht enthält, 7.000 bis 13.000 mPas beträgt.

## Claims

1. Use of a membrane filter for regenerating used dialysate, comprising a plurality of hollow fiber membranes prepared by the following method:
a) providing two spinning mass solutions A and B, wherein the spinning mass solution A is a solution of an esterified cellulose for the lumen-side selection layer and the spinning mass solution B is a solution containing a polymer for the support layer selected from the group consisting of polyvinylpyrrolidone (PVP), polyethersulfone (PES), polyetherimide (PEI), polyamide (PA), polycarbonate (PC), polystyrene (PS), polymethylmethacrylate (PMMA), polyvinylidene fluoride (PVDF), polyacrylonitrile (PAN), polyimide (PI), polysulfone (PSU), and/or polyurethane (PU) and mixtures thereof,
b) setting the precipitation bath temperature at 40 to 95°C,
c) bringing the spinning mass solutions A and B into contact with water as internal precipitant via a hollow fibre spinneret,
(d) coagulating and precipitating the extrudate consisting of the substances dissolved in the spinning mass solutions A and B, the spinning block temperature being set at 5 to 40°C,
wherein the hollow fiber membranes comprise a coextrudate of a support layer and a lumen-side selection layer, wherein the selection layer consists of acetyl cellulose and the degree of acetylation lies in the range of 0.5 to 3, and wherein the thickness of the selection layer is less than 800 nm.

2. Use of a membrane filter for regenerating used dialysate, comprising a plurality of hollow fiber membranes according to claim 1, **characterized in that** the thickness of the selection layer is 100 nm to less than 800 nm.

3. Use of a membrane filter for regenerating used dialysate, comprising a plurality of hollow fiber membranes according to claim 1 to 2, **characterized in that** the urea permeability is between 10 and 80 g per day per m² and the sodium permeability is between 0 and 112 mmol per day per m², measured at a flow rate of 50 ml/min against a glucose solution of 538 mM at room temperature after 2 hours of circulation, wherein 500-700 ml of a urea-containing salt solution, which contains 25 mM urea, 141 mM NaCl, 2.5 mM CaCl₂, 249 mM glucose and which is recirculated lumen-side through the hollow fibre at 50 ml/min, is used to determine the urea and salt permeability, and wherein on the outside of the membrane a 538 mM glucose solution is pumped in contraflow with a flow rate of 50 ml/min.

4. Use of a membrane filter for regenerating used dialysate, comprising a plurality of hollow fiber membranes according to any one of claims 1 to 3, **characterized in that** the material of the support layer is selected from polyvinylpyrrolidone (PVP), polyethersulfone (PES), polyetherimide (PEI), polyamide (PA), polycarbonate (PC), polystyrene (PS), polymethylmethacrylate (PMMA), polyvinylidene fluoride (PVDF), polyacrylonitrile (PAN), polyimide (PI), polysulfone (PSU), and/or polyurethane (PU) and mixtures thereof.

5. Use of a membrane filter for regenerating used dialysate, comprising a plurality of hollow fiber membranes according to claim 4, **characterized in that** the material of the support layer is selected from polysulfone (PSU), polyvinylpyrrolidone (PVP) and mixtures thereof.

6. Use of a membrane filter for regenerating used dialysate, comprising a plurality of hollow fiber membranes according to any one of claims 1 to 5, **characterized in that** the thickness of the support layer lies in the range of 20 to 50 µm, preferably in the range of 30 to 40 µm.

7. Use of a membrane filter for regenerating used dialysate, comprising a plurality of hollow fiber membranes according to one of the preceding claims, **characterized in that** the spinning speed is 200 to 400 mm/s.

8. Use of a membrane filter for regenerating used dialysate, comprising a plurality of hollow fiber membranes according to one of the preceding claims, **characterized in that** the viscosity of the spinning mass solution A, which contains cellulose acetate, is 10000 to 17000 mPas.

9. Use of a membrane filter for regenerating used dialysate, comprising a plurality of hollow fiber membranes according to one of the preceding claims, **characterized in that** the viscosity of the spinning mass solution B, which contains the polymer for the support layer, is 7000 to 13000 mPas.

## Revendications

1. Utilisation d'un filtre à membrane pour la régénération de dialysat utilisé, comprenant une pluralité de membranes à fibres creuses préparées selon le procédé suivant:
a) fournir deux solutions de masse de filage A et B, dans lesquelles la solution de masse de filage A est une solution d'une cellulose estérifiée pour la couche de sélection côté lumière et la solution de masse en rotation B est une solution contenant un polymère pour la couche de support choisie parmi le groupe constitué de polyvinylpyrrolidone (PVP), polyéthersulfone (PES), polyétherimide (PEI), polyamide (PA), polycarbonate (PC), polystyrène (PS), polyéthylméthacrylate (PMMA), polyfluorure de vinylidène (PVDF), polyacrylonitrile (PAN), polyimide (PI), polysulfone (PSU) et/ou polyuréthane (PU) et leurs mélanges,
b) régler la température du bain de précipitation entre 40 et 95°C,
c) mettre en contact des solutions de masses de filage A et B avec de l'eau en tant que précipitant interne via une filière à fibres creuses,
(d) coaguler et précipiter l'extrudat constitué des substances dissoutes dans les solutions de masses de filage A et B, la température du bloc de filage étant réglée entre 5 et 40°C,
dans laquelle les membranes à fibres creuses comprennent un coextrudat d'une couche de support et une couche de sélection côté lumière, la couche de sélection étant constituée d'acétate de cellulose et le degré d'acétylation étant compris dans la plage de 0,5 à 3, et dans laquelle l'épaisseur de la couche de sélection est inférieure à 800 nm.

2. Utilisation d'un filtre à membrane pour la régénération de dialysat utilisé, comprenant une pluralité de membranes à fibres creuses selon la revendication 1, **caractérisé en ce que** l'épaisseur de la couche de sélection est comprise entre 100 nm et moins de 800 nm.

3. Utilisation d'un filtre à membrane pour la régénération de dialysat utilisé, comprenant une pluralité de membranes à fibres creuses selon les revendications 1 à 2, **caractérisé en ce que** la perméabilité à l'urée est comprise entre 10 et 80 g par jour et par m² et la perméabilité au sodium est comprise entre 0 et 112 mmol par jour et par m², mesurés à un débit de 50 ml/min contre une solution de glucose de 538 mM à la température ambiante après 2 heures de circulation, dans laquelle 500 à 700 ml d'une solution de sel contenant de l'urée, 25 mM de l'urée, 141 mM de NaCl, 2,5 mM de CaCl₂ et 249 mM de glucose et qui est recyclé côté lumière à travers la fibre creuse à 50 ml/min, est utilisé pour déterminer la perméabilité à l'urée et au sel, et dans laquelle une solution de 538 mM de glucose est pompée à la côté extérieure de la membrane à contre-courant avec un débit de 50 ml/min.

4. Utilisation d'un filtre à membrane pour la régénération de dialysat utilisé, comprenant une pluralité de membranes à fibres creuses selon l'une quelconque des revendications 1 à 3, **caractérisé en ce que** le matériau de la couche support est choisi parmi le polyvinylpyrrolidone (PVP), le polyéthersulfone (PES), le polyétherimide (PEI), le polyamide (PA), le polycarbonate (PC), le polystyrène (PS), le polyméthacrylate de méthyle (PMMA), la polyfluorure de vinylidène (PVDF), le polyacrylonitrile (PAN), le polyimide (PI), le polysulfone (PSU), et/ou le polyuréthane (PU) et leurs mélanges.

5. Utilisation d'un filtre à membrane pour la régénération de dialysat utilisé, comprenant une pluralité de membranes à fibres creuses selon la revendication 4, **caractérisé en ce que** le matériau de la couche de support est choisi parmi le polysulfone (PSU), le polyvinylpyrrolidone (PVP) et leurs mélanges.

6. Utilisation d'un filtre à membrane pour la régénération de dialysat utilisé, comprenant une pluralité de membranes à fibres creuses selon l'une quelconque des revendications 1 à 5, **caractérisé en ce que** l'épaisseur de la couche de support est comprise entre 20 et 50 µm, de préférence dans la plage de 30 à 40 µm.

7. Utilisation d'un filtre à membrane pour la régénération de dialysat utilisé, comprenant une pluralité de membranes à fibres creuses selon l'une des revendications précédentes, **caractérisé en ce que** la vitesse de rotation est de 200 à 400 mm/s.

8. Utilisation d'un filtre à membrane pour la régénération de dialysat utilisé, comprenant une pluralité de membranes à fibres creuses selon l'une des revendications précédentes, **caractérisé en ce que** la viscosité de la solution de la masse de filage A, qui contient de l'acétate de cellulose, est comprise entre 10 000 et 17 000 mPas.

9. Utilisation d'un filtre à membrane pour la régénération de dialysat utilisé, comprenant une pluralité de membranes à fibres creuses selon l'une des revendications précédentes, **caractérisé en ce que** la viscosité de la solution de la masse de filage B, qui contient le polymère pour la couche de support, est 7000 à 13000 mPas.
